# EUROPEAN PATENT APPLICATION

(11) **EP 0 819 435 A1**
(43) Date of publication of application: **21.01.1998**
(21) Application number: 96202021.0
(22) Date of filing: 16.07.1996
(51) Int. Cl.: A61K 39/395, A61K 39/40, A61K 39/42, A61K 9/107, A23K 1/18

(54) **Passive immunisation of fish and shell fish and immunoglobulin emulsions used for it**

(71) Applicant: K.U. LEUVEN RESEARCH & DEVELOPMENT, 3000 Leuven (BE)
(72) Inventor: Roelants, Ivo, 3000 Leuven (BE); Grisez, Luc Lodewijk Jules, 3390 Tilt-Winge (BE); Ollevier, Frans, 3212 Lubbeek (BE)
(74) Representative: Van Someren, Petronella F. H. M.

(57) **Abstract**

The invention relates to small aquatic animals having taken up one or more bioactive agents for use in the treatment or prophylaxis of animals, in particular fish and shellfish. The bioactive agents are for example immunoglobulins and the treatment consists of passive immunisation of fish and shellfish larvae. The small aquatic animals are for example zooplankton, such as species of the genus Artemia, rotifera and/or copepoda.

The invention further relates to a delivery form for administering one or more bio-active agents to small aquatic animals in particular live zooplankton, comprising a blend of one or more edible oils; one or more bio-active agents, and one or more emulsifiers.

## Description

The present invention relates to a vehicle comprising a biologically active (bioactive) agent for use in the treatment and prophylaxis of animals, in particular fish and shellfish. The invention in particular relates to a vehicle comprising immunoglobulins for use in the passive immunisation of animals, in particular fish and shellfish and their larvae. The invention further relates to a delivery form for use in the preparation of the vehicle and to various methods for preparing both the vehicle and the delivery form.

The development of intensive culture of fish and shellfish has yielded an increased demand for larval and juvenile post larval organisms. This increased demand could well be met by the fish and shellfish nurseries that were established. However, intensification in the fish and shellfish larval production resulted in a dramatic increase in the incidence of microbial disease (Sorgeloos & Léger, 1992)

For example, marine hatcheries struggle with large mortalities of fish larvae due to bacterial diseases such as vibriosis, which leads to a low and expensive yield. Grisez et al. (1995) demonstrated for example that the marine fish pathogen Vibrio anguillarum is responsible for large mortalities of European sea bream and sea bass. The origin of the infection appears to be the live food (zooplankton, like rotifers and Artemia) that is still essential during the first days of feeding larvae.

Presently the treatment of such microbial infections, like Vibrio, is often carried out by delivering antibiotics directly to the culture water or by injection with antibodies carried out by a veterinarian (passive immunisation).

However, there are several problems related to these methods such as discharge of antibiotics into the environment and development of resistant bacterial strains, which can lead to hazardous ecological effects.

Furthermore veterinary immunisation is inefficient, being time consuming, difficult to carry out and hazardous to the fish.

It is therefore a first object of the present invention to provide a vehicle for introducing one or more bioactive agents into animals such as fish larvae whereby one or more of the above problems are solved and by which the bioactive agent(s) remain substantially intact before reaching their destination in the animal.

According to the invention this is achieved in two steps. First, small aquatic animals, mainly small crustaceans, collectively called zooplankton, are fed with the bioactive agent. Thus, the vehicle comprising the bioactive agent is prepared. Then, the animals to be treated are fed with the small aquatic animals that have taken up the bioactive agent. Thus, the invention in a first aspect thereof provides for small aquatic animals, such as zooplankton, having taken up one or more biologically active agents as a vehicle for use in the treatment or prophylaxis of animals, in particular fish and shellfish larvae.

The incorporation of the bioactive agent in the zooplankton is possible since zooplankton are non-selective filter feeders. However, simply adding the bioactive agent to the water in which the zooplankton are present would result in a substantial loss in bio-activity. Therefore, according to a second aspect of the invention, the uptake of the bioactive agent is preferably achieved by means of a delivery form for administering one or more bioactive agents to small aquatic animals, in particular live zooplankton, comprising a blend of the following constituents:
a) one or more edible oils;
b) one or more bio-active agents, and
c) one or more emulsifiers.
The delivery form optionally further comprises water as another constituent, and is then in the form of an emulsion or cream obtainable by emulsifying the blend of constituents a), b) and c) with the water. The emulsion is a water-in-oil emulsion. The cream is also a water-in-oil emulsion with 10-20% water but more viscous than the emulsion. The cream is the preferred embodiment which after adding to an excess of water (e.g. the culture water of zooplankton) will become an oil-in-water micro-emulsion consisting of small droplets that can be taken up by the small zooplankton. The emulsion and cream are oil-in-water type emulsions.

As an alternative the delivery form may take the form of a powder (or micro-particulate material), obtainable by absorbing the blend of the constituents a), b) and c) and/or the emulsion or cream of the blend and water onto a carrier, adding a gellable binder, inducing gelling of the binder, and drying and grinding the gel. The powder thus obtained may be fed to the small aquatic animals as such.

In order to obtain the powder delivery form the emulsion or cream is preferably prepared with a water fraction containing 10% sodium alginate as a binder, wherein sodium alginate is about 4 to 10% of the oil fraction. The water phase may be 40% to 100% of the oilfraction. This mixture is subsequently preferably warmed up to for example 80°C whereafter it is mixed with a hydrophilic silica carrier with an average particle size preferably of less than 100 nm. As an alternative, this inert silicate carrier may be replaced partially or totally by a protein carrier.

After absorption of the emulsion on the carrier this blend is abruptly cooled to induce gelling, whereafter the product is dried and finally ground to a particle size (for example in the range of 1-20 µm) suitable for uptake by Artemia or rotifer.

As an alternative, the paste obtained after adsorption of the creamy emulsion on the carrier is extruded into a solution of 0.5-10% CaCl₂ in water to evoke instant gelling after which the gel is dried and ground. Warming the mixture may then be omitted.

"Delivery form" as used herein is intended to encompass any composition comprising the three above identified constituents in some form or other. The delivery form may thus be a liquid or solid feed for small aquatic animals etc..

The oil used in the delivery form may for example be edible fish or vegetable oils, such as marine oils, such as cod oil, cod liver oil, pollack oil, squid oil, shark liver oil, or vegetable oils, such as sesame oil, sunflower oil, almond oil, safflower oil etc..

The delivery form of the invention is suitable for administering all kinds of bioactive agents, provided they are soluble in an oil. Water-soluble bioactive agents may be incorporated in the emulsion but will diffuse as soon as the delivery form is administered to the culture water of the fish or shellfish. This will substantially decrease the efficacy thereof. Therefore, a preferred embodiment of the bioactive agent(s) is (are) any substance(s) that may be incorporated into an oily phase. Surprisingly it was found according to the invention that it is possible to target (specific) immunoglobulins or antibodies to living feed without loss of activity by using the emulsion or microparticles.

The terms "immunoglobulins" or "antibodies" as used herein are intended to encompass both monoclonal and polyclonal antibodies, both in isolated or in non-isolated form, such as an antiserum, egg yolk of actively immunised birds, or the milk of actively immunised mammals.

The emulsifier may be selected from the group consisting of lecithin, polyglycerol fatty acid, sorbitan fatty acid, sodium oleate, polyoxyethylene fatty acid ester, polyoxyethylene fatty glyceride, polyoxyethylene fat, and anhydrous maltose or may be a combination of one or more of these.

The delivery forms may further comprise various additives, such as one or more preservatives, for example selected from the group consisting of benzoic acid, sorbic acid, propionic acid, or their salts, and/or antibacterial agents, such as protamine sulphate or rosemary extract. For a stronger preservative effect the water can be acidified, for example by means of ascorbic acid, acetic acid, lactic acid or citric acid. Preservatives are intended to protect the delivery form against bacterial or fungal contamination.

To avoid foaming of the delivery form upon contact with water when feeding the zooplankton it may further comprise one or more anti-foaming agents, for example a silicon polymer.

Under extreme conditions of temperature or oxygenation oxidation of the oil may occur which will then become rancid. This may be avoided by incorporating into the delivery form one or more oxygen removal agents, for example selected from the group consisting of glucose-oxidase, ascorbic acid or salts thereof. Other anti-oxidation agents that work via a different mechanism comprise for example butylated hydroxyanisole (BHA), butylated hydroxytoluene, (BHT),L-tocopherol, propyl gallate, carotinoids, and vitamin C palmitate.

The above delivery form proved acceptable for the uptake of bioactive agents at high transfer rate by the live food source organism, plankton, especially Artemia, rotifers and copepoda, which are non selective filter feeders, whereby the bioactive agents retained bioactivity.

The invention further relates to a method of preparing a delivery form of the invention, comprising blending the constituents a), b) and c), optionally together with other additives. For preparing an emulsion or cream the method of the invention, comprises blending the constituents a), b) and c), optionally together with other additives and emulsifying the so obtained blend in water.

In another aspect the invention provides a method of preparing a micro-particulate (powder) delivery form, comprising blending the constituents a), b) and c), optionally together with other additives, optionally emulsifying the so obtained blend in water to obtain an emulsion or cream, adsorbing the blend, emulsion or cream onto a carrier material, adding a gellable binder to the carrier, gelling the mixture of the carrier and binder and drying and grinding the gel.

The delivery form of the invention may be used to prepare a preparation for oral uptake for treatment and/or prophylaxis of animals, in particular fish and shellfish, with a bio-active agent, such as one or more specific immunoglobulins. The preparation then comprises small aquatic animals in particular zooplankton, to which the bio-active agent was administered by means of a delivery form of the invention, optionally in the presence of a suitable excipient. Preferably, the treatment consists of passive immunisation of fish and shellfish larvae and the bioactive agent comprises immunoglobulins, for example directed against bacterial, protozoan or viral pathogens, such as Vibrio anguillarum.

The invention thus also provides for small aquatic animals having taken up one or more bio-active agents, for use in the treatment and/or prophylaxis of animals, in particular fish and shellfish. The small aquatic animals are preferably zooplankton, such as species of the genus Artemia, rotifera and/or copepoda.

According to the invention the small aquatic animals may be prepared by feeding them with a delivery form of the invention.

The invention also relates to the use of small aquatic animals having been fed with a delivery form of the invention for preparing a preparation for the treatment or prophylaxis of animals, such as fish or shellfish.

The invention will now be further elucidated by way of the following examples, which are given for illustration purposes only.

### EXAMPLES

### EXAMPLE 1

### Formulation of the delivery form

The following shows the preparation of a base formulation for the various types of delivery form, namely blend, emulsion, cream and powder (micro-particulate). The bioactive agent is not included in the base formulation. Example 2 will indicate which bioactive agents are used in what amount and how they were prepared.

### 1. Blend

120 parts of a refined edible fish oil (marine oil EPAX 5500 TG (Pronova Biocare)) were blended with an emulsifier consisting of a mixture of 1.86 parts of polyglycerol fatty acid, 3.6 parts of polyoxyethylene fatty acid ester and 1 part of polyoxyethylene fatty glyceride. The mixture of oil and emulsifier thus obtained constitutes what is referred to as "blend".

The emulsifier used herein is only one example of suitable emulsifiers. The invention does not reside in a particular emulsifier and the skilled person will be capable of choosing alternatives without departing from the scope of the invention.

### 2. Cream

A cream according to the invention can be obtained by mixing the blend with 10-20% water on the total. Mixing was achieved by homogenisation using an ultrturax mixer or Bambury kitchen mixer at room temperature (18°C) and gradually adding the "blend" obtained under 1. to the water phase. Mixing was continued until the cream was formed.

### 3. Oil-in-water micro-emulsion

The cream obtained as under 2. was mixed with filtered sea water at 26°C, at a concentration up to 2 gram cream per litre water.

For practical use the emulsion must be stable (i.e. should not separate into the aqueous and oily phase) for at least 48 hours at a temperature range from 16°C up to 32°C, in the presence of suspended particles, in particular live zooplankton in suspension, and occasional strong aeration.

### 4. Powdered delivery form

In order to obtain a powdered delivery form, the blend was either first emulsified with 10% water to yield a stable cream. As an alternative the blend was used as such.

The cream was then (further) emulsified in water containing 10% sodium alginate as a binder and 0.1% of the sequestrate, sodium hexametaphosphate, which prevents that sodium alginate will gel immediately upon mixing. In the final emulsion the amount of sodium alginate is about 4 to 10% of the oil fraction. The water phase may be 40% to 100% of the oil fraction.

Subsequently this emulsion was intensively mixed with a silica (Aerosil, Degussa) carrier with an average particle size of less than 100 nm. As an alternative another hydrophilic silica may be used or can be replaced partially or totally by a protein based oil carrier, such as casein, whey protein, soy protein etc.. After absorption of the creamy emulsion on the carrier a paste was obtained which was extruded into a solution of 0.5% to 10% CaCl₂ solution to evoke instant gelling. The resulting product was dried and finally ground to a particle size (about 4 µm) suitable for uptake by Artemia or rotifer.

As an alternative the emulsion of the blend or cream with water was heated to 80°C and subsequently mixed with the Aerosil. After absorption of the emulsion on the carrier the mixture is instantly cooled to induce gelling. After that the gel thus obtained was dried and ground. By means of a vibrator sieve a fraction of the desired particle size, for example in the range of 1-20 µm, was separated off.

### EXAMPLE 2

### Preparation of bioactive agent and incorporation in the delivery form

### 1. Immunoglobulins

In the remainder of the examples various immunoglobulins (antibodies, antisera) are used as the bioactive agent. The preparation of these are described in the following.

### 1.1. Anti-cf-GH antiserum

In the examples 3 and 4 an antiserum against catfish growth hormone (cf-GH) was used as the bioactive agent. The antiserum was prepared in female New Zealand white rabbits by by intramuscular injection of cf-GH every three weeks during a period of 30 weeks.

### 1.2. Anti-Vibrio antiserum

In example 5 antibodies against Vibrio anguillarum were used. Vibrio anguillarum was chosen as pathogen since this bacterium is responsible for severe septicaemic infections in virtually all cultured marine fish species (Toranzo and Barja, 1993). Only two serotypes of this species are responsible for most infections in fish: serotype O1 and serotype O2 (Larsen et al., 1994). An antiserum was produced against the lipopolysaccharide antigens (LPS or O antigens) of a serotype O2 strain (Grisez and Ollevier, 1995). LPS is the main antigen for this species (Toranzo and Barja, 1993).

An antiserum was produced against an O-antigen suspension of Vibrio anguillarum serotype O2a (Grisez and Ollevier, 1995) strain NCIMB 6. Bacteria grown overnight on brain heart infusion agar (Difco, Detroit, Michigan, USA) supplemented with 1% NaCl (w/v) (BN), were harvested in sterile phosphate buffered saline (PBS; 50 mM phosphate buffer, 0.15 M NaCl (pH 7.2)) to which formaldehyde was added to a final concentration of 0.3% (v/v). The suspension was heated for 1 h at 100°C in a warm water bath, and bacterial O antigens were harvested, washed and subsequently resuspended in sterile PBS to an optical density of 0.7 (A₆₀₀). Aliquots (1 ml) of the resulting O-antigen suspension for immunisation were frozen at -20°C until use.

Polyclonal antisera to this strain were raised in New Zealand white rabbits by repeated injections of O-antigen suspensions. Animals were given subcutaneous injections of 0.5 ml of O-antigen suspension mixed with an equal volume of Freund's incomplete adjuvant (Difco, Detroit, Michigan, USA). Three weeks later, intravenous injections of 0.25, 0.5, 1 and 1 ml of O-antigen suspension without Freund's incomplete adjuvant were given at daily intervals. One week after the last injection, animals were exsanguinated by cardiac puncture, the blood was allowed to clot overnight at 5°C, and the antisera were stored at -20°C.

### 2. Incorporation into delivery form

For examples 3 and 4 the anti-cf-GH antiserum was added to the cream by intensive mixing in an ultra-turax homogenizer in an amount of 1 ml rabbit anti-cf-GH antiserum per 5 ml of cream.

The preparation used in example 5 was based on a micro-particulate delivery form (powder). The passive vaccine was incorporated into this delivery form by adding the antiserum (instead of water) at a ratio of 1/5 to the blend of oil and emulsifiers, as given in Example 1, under 1.. After emulsification a micro-particulate delivery form was produced as described above in example 1 under 4..

### EXAMPLE 3

### Uptake of bioactive agent by zooplankton

First the uptake of bioactive agent by zooplankton was studied in a model using cf-GH as the bioactive agent.

A cream prepared as described in example 2 was fed to newly hatched Artemia nauplii (300,000 animals per liter filtered sea water of 26°C, more than 4 ppm oxygen) or to the rotifers Brachionus plicatilus (400,000 animals per liter filtered sea water at 22°C, more than 4 ppm oxygen). Artemia was fed 600 mg cream, the rotifers received 200 mg.

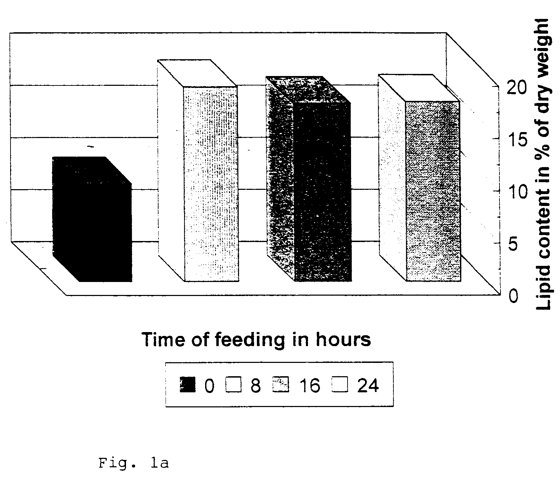

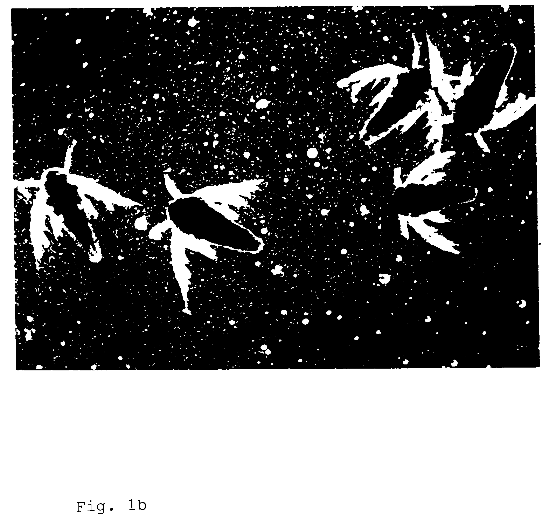

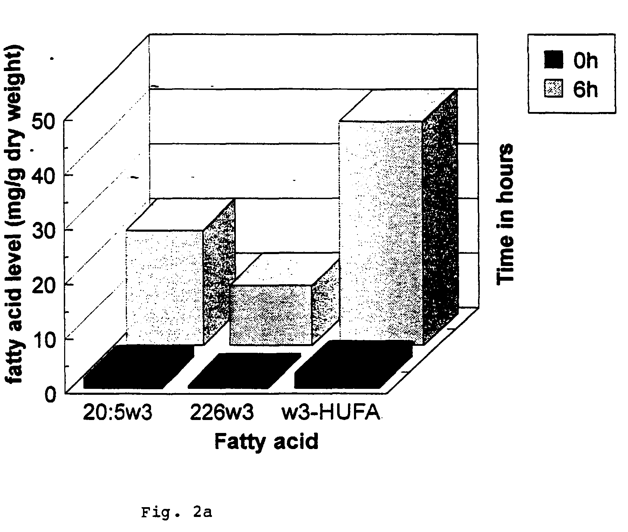

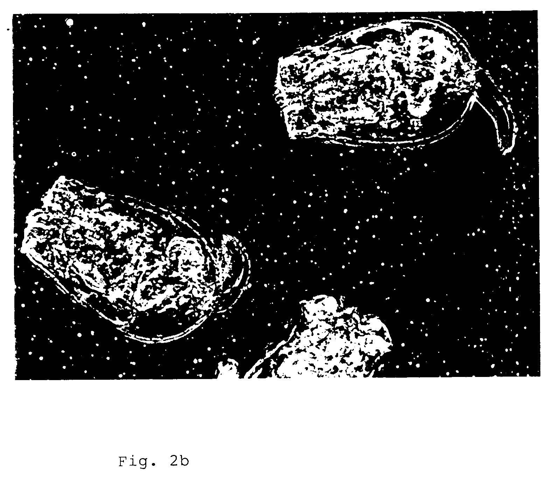

The cream formed a stable emulsion in the zooplankton culture water and the oil droplets were readily taken up by both species as is shown in figs. 1a and 1b, and 2a and 2b, respectively. Fig. 1a shows the lipid content in Artemia at 0, 8, 16 and 24 hours after feeding. Fig. 2a shows a clear uptake in rotifer of the cream which has the marine oil (fish oil) as major component.

### EXAMPLE 4

### Presence of cf-GH in zooplankton

### 1. Radioimmunoassay

Example 3 demonstrated that the oil from the emulsion was taken up by the zooplankton. This example is intended to illustrate that with the oil the zooplankton actual take up the bioactive agent.

Purified catfish growth hormone (cf-GH) was iodinated according to the Iodo-gen method,(The purification and physico-chemical characterization of this hormone have been described by Lescroart et al, 1995). After purification of the iodinated mixture by gel permeation, (Sepharose G-25, Pharmacia Fine Chemicals) the fraction containing the iodinated cf-GH was determined in a γ-counter (Canberra Packard, Zellik, Belgium), and diluted in RIA buffer (5 mM NaH₂PO₄, 5 Mm Na₂HP0₄, 0.9% NaCL and 1% BSA, pH 7.6).

Newly hatched Artemia nauplii were transferred at a density of 300,000 per litre to 1.5 litre tanks. They were kept on artificial seawater with the anti-cf-GH antiserum enriched (1 ml antiserum/5 ml cream) cream (1 gram per litre culture water) of the blend prepared under 1.. The incubation tanks were aerated sufficiently to keep the nauplii in suspension and the oxygen levels above 4 ppm.

After allowing the Artemia to feed for 6 hours, these were collected on a 120 µm screen and rinsed. Weighed samples of Artemia were homogenised and centrifuged, at 10,000 rpm and 4°C, and a dilution range of the supernatants was used in a radioimmunoassay to establish a binder-dilution curve of the antiserum to the antigen. As a control Artemia fed with the emulsion without the anti-serum, and Artemia fed with antiserum without emulsion were used.

Dilution series of test species and control, ranging from 1/100 to 1 x 10⁻⁶ were made in RIA buffer. 100 µl of these dilutions were incubated for 24 hr with 100 µl of the iodinated GH at a concentration of 10,000 cpm in RIA buffer containing 1% BSA. To precipitate the antigen-antibody complex a second antibody, 50 µl donkey anti-rabbit (Sac-cell), was then added and incubated during 30 minutes. One ml of 5% EDTA solutions was added and the complex was pelleted by centrifugation at 3000 rpm for 20 min.

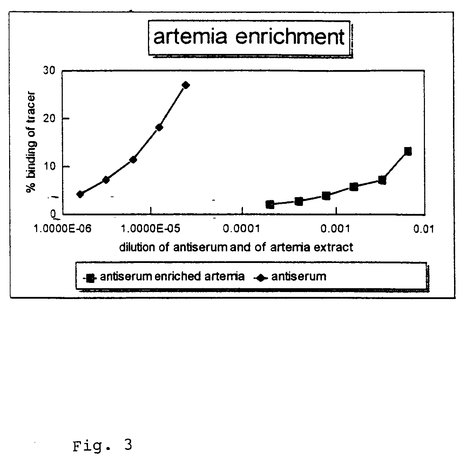

After removal of the supernatant the precipitate was counted in a gamma-counter, and the dilution curves plotted (fig. 3).

The binder-dilution curve of Artemia fed on the antiserum enriched emulsion, indicated the presence of antibodies directed against cf-GH, which demonstrates the transfer of these immunoglobulins from the incubation medium into the feeding Artemia nauplii. The amount of antibodies in the control that received the antiserum without the emulsion was much lower. The control Artemia that were fed the oil emulsion without antiserum did not display binding with the tracer.

### 2. Immunoblotting

The Artemia fed for six hours on the cream enriched with the anti-serum, were also subjected to immunoblotting to determine presence of the anti-serum in the Artemia.

Purified cf-GH was subjected to sodium dodecyl sulphate (SDS) PAGE at pH 6.4 using homogenous gels in the Phast System of Pharmacia Fine Chemicals (Brussels). A 2 µl sample, containing 10-50 ng of protein, was electrophoresed. After this procedure the proteins were blotted onto Immobilon transfer membranes (Millipore, Brussels, Belgium).

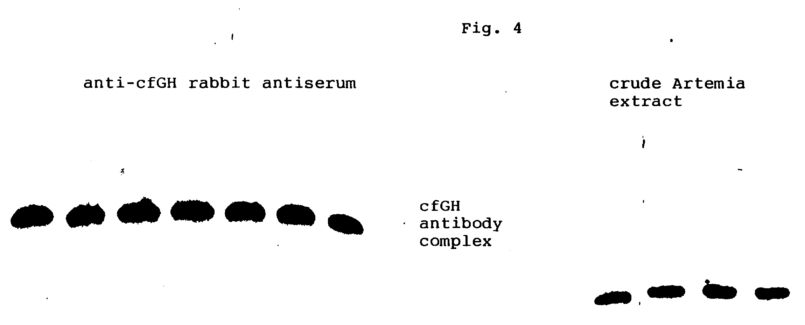

A membranes was either immunostained with the antiserum or with the Artemia extract (diluted 1/100). Alkaline phosphatase-conjugated second antibodies (Dakopatts, Gent, Belgium; dilution 1/200) and nitro blue tetrazolium and bromo-chloro-indolyl-phosphate (Blake et al., 1984) were used to revealed the protein-antibody complexes between cf-GH and anti-cf-GH formed on the membrane. The results are shown in Fig. 4.

The control rabbit antiserum and the extract of Artemia priory fed with anti-cf-GH antiserum oil emulsion clearly stained the band on the immunoblots of affinity-purified cf-GH. This indicates accumulation of bioactive immunoglobulines in the Artemia nauplii.

### 3. In situ immunostaining

A sagittal section of 7 µm of the African catfish pituitary was immunostained with the extract of Artemia fed, as above, with anti-cf-GH antiserum enriched (1 ml antiserum/5 ml cream) cream.

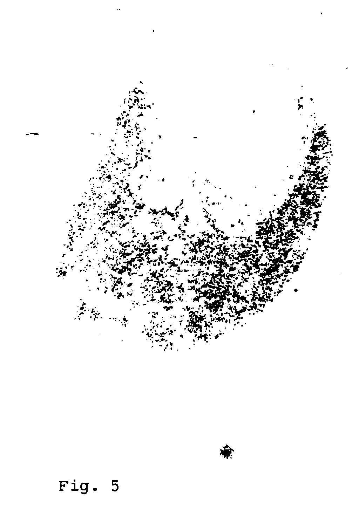

Incubation with the extract (1/100) positively stained the GH-cell in the proximal pars distalis of the pituitary (Fig. 5). Incubation with a control artemia extract did not stain the adjacent pituitary section (data not shown).

### EXAMPLE 5

### Transfer of a passive vaccine against Vibrio anguillarum to zooplankton

Following the above, further experiments were carried out to investigate the transfer of a passive vaccine raised against a typical fish pathogen, Vibrio anguillarum serotype 02a (Grisez and Ollevier, 1995) strain NCIMB 6, to zooplankton.

### 1. Materials and methods

### 1.1. Delivery form and bioactive agent

An antiserum raised against serotype O2 of the marine fish pathogen Vibrio anguillarum was incorporated in a micro-particulate Artemia feed. The feed was prepared as described in Example 2 under 2.

### 1.2. Feeding of Artemia with the passive vaccine

For the study of antibody transfer kinetics the newly hatched Artemia nauplii were transferred at a density of 300 000 nauplii per litre to 1.5 litre tanks. They were kept on artificial seawater with the micro-particulate delivery form (1 gram per litre) to which the passive vaccine against Vibrio anguillarum serotype 02a (Grisez and Ollevier, 1995) strain NCIMB 6 was added. These incubation tanks were aerated sufficiently to keep the nauplii in suspension and the oxygen levels above 4 ppm.

After 2 hours of feeding the nauplii were harvested on a 120 µm screen and thoroughly rinsed with artificial seawater and quickly with tap water just prior to snap freezing (freezing by contact with very low temperatures) for storage at -80°C.

### 1.3. Extraction of Artemia

Weighed samples of Artemia were mixed 1/1 in an assay buffer (50 mM Tris, 1% BSA + 0.05 Azide (to avoid fungal or bacterial infection, 0.1% Triton X, pH 7.8). Then they were homogenized under ultra-turrax and centrifuged at 10,000 rpm and 4°C. The supernatant was removed and vigorously mixed with CH₂Cl₂ (2/5 v/v). The upper water layer was removed and this extraction method was repeated once more.

Finally the water phase, hereof referred to as "anti-Vibrio Artemia extract", was blast frozen and stored at -80°C until assaying.

### 1.4. Purification of lipopolysaccharides (LPS) from V. anguillarum strain NCIMB 6

LPS was prepared by the proteinase K method described by Hichcock and Brown (1983). Bacteria, harvested and washed in sterile PBS, were resuspended in sterile PBS to a transmission of 30% at 525 nm. The cell pellet obtained from 1.5 ml of this suspension was resuspended in 50 µl of Laemmli sample buffer (Laemmli, 1970) and heated for 10 min at 100°C. After heating, the residual bacterial debris was removed by centrifugation (8 min at 13,000 x g) and the supernatant was transferred into a new Eppendorf tube. An aliquot (10 µl) of proteinase K solution (2.5 µg of proteinase per µl of sample buffer) was added to the sample, and the mixture was incubated for 30 min at 60°C. This proteinase K treatment was performed twice. The resulting LPS samples were stored at -20°C.

### 1.5 SDS-PAGE and Western blotting

SDS-PAGE of LPS samples for Western blotting (immunoblotting) were performed on the Phast System (Pharmacia LKB, Uppsala, Sweden) with 12.5% (w/v) acrylamide homogeneous precast gels. LPS samples for electrophoresis and subsequent blotting, prepared as discussed above, were diluted 1:4 in sample buffer. Electrotransfer of the LPS to polyvinylidine difluoride membranes (Bio-Rad) was out in the Phast Transfer semidry electrophoretic transfer system (Pharmacia LKB). Blots were blocked for 2 h with skimmed milk in 50 mM Tris buffer (pH 7.6), washed in Tris saline buffer (TS; 10 mM Tris buffer, 150 mM NacL, 0.1% (v/v) Triton X-100 (pH 7.6)), and subsequently incubated overnight in the presence of the primary antibody.

As primary antibody, either the polyclonal antiserum was used in a dilution 1:2,500 in TS (positive control), or an extract of Artemia that were fed an anti-Vibrio delivery form, diluted 1:4 in TS (test condition) or an extract of Artemia not fed with an antiserum enriched delivery form, diluted 1:4 in TS (negative control).

The secondary antibody, alkaline phosphatase-conjugated goat anti-rabbit antibody (Dakopatts, Copenhagen, Denmark), diluted 1:400 in TS, was applied for 1 h, and the blots were developed as described by Blake et al. (1984) in a substrate solution containing BCIP (5-bromo-4-chloro-3-indolyl phosphate, p-toluidine salt (Sigma, St. Louis, Mo.)) and nitroblue tetrazolium chloride (Sigma) in alkaline phosphate buffer (100 mM Tris buffer, 100 mM NaCl, 5 mM MgCl2 (pH 9.6)).

### 2. Results

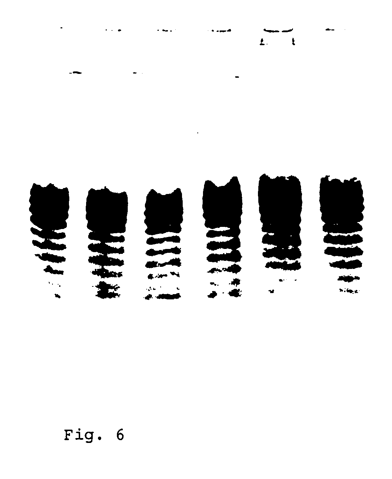

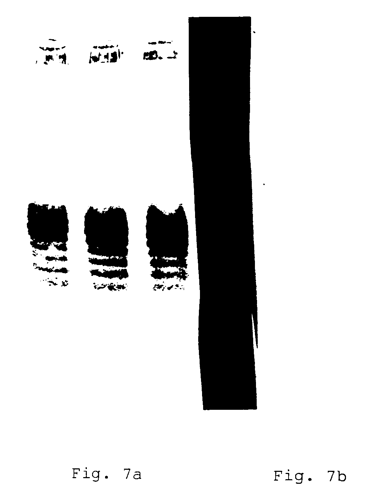

The results of the LPS blotting with antiserum against Vibrio anguillarum and with control Artemia extract are demonstrated in figures 6 and 7. The test condition in which the purified, PAGE separated and subsequently Western blotted LPS of V. anguillarum was developed with the extract of Artemia, fed with the antiserum enriched micro-particulate deliver form, is presented in figure 6. The typical, serotype O2, LPS banding pattern obtained, is exactly the same as the pattern obtained with the antiserum in pure form (positive control, Fig. 7A). The negative control, in which the second half of the Western blot used for the controls was developed with an extract of Artemia which had been fed the same micro-particulate delivery form as the test condition but to which no antiserum was added, shows no LPS banding pattern (Fig. 7B). The negative control proves that no endogenous LPS specific action is present in the micro-particulate deliver form or in the Artemia.

The results demonstrate that the antiserum, directed against the major antigens of V. anguillarum O2, remains bio-active and recognizes these antigens after incorporation into the micro-particulate delivery form, after it has been taken up by the Artemia, and after subsequent extraction of the Artemia.

## Claims

1. Small aquatic animals having taken up one or more bioactive agents for use in the treatment or prophylaxis of animals, in particular fish and shellfish.

2. Small aquatic animals as claimed in claim 1, wherein the bioactive agents are immunoglobulins and the treatment consists of passive immunisation of fish and shellfish larvae.

3. Small aquatic animals as claimed in claim 2, wherein the immunoglobulins are directed against bacterial, protozoan or viral infectious agents, such as Vibrio anguillarum.

4. Small aquatic animals as claimed in claim 1, 2 and 3, which small aquatic animals are zooplankton, such as species of the genus Artemia, rotifera and/or copepoda.

5. Use of small aquatic animals as claimed in claims 1-4 for the preparation of a pharmaceutical composition for the treatment or prophylaxis of animals, in particular fish and shellfish larvae.

6. Delivery form for administering one or more bio-active agents to small aquatic animals as claimed in claims 1-4, in particular live zooplankton, comprising a blend of the following constituents:
a) one or more edible oils;
b) one or more bio-active agents, and
c) one or more emulsifiers.

7. Delivery form as claimed in claim 6 further comprising water as another constituent, and being in the form of an emulsion or cream obtainable by emulsifying the blend of constituents a), b) and c) with the water.

8. Delivery form as claimed in claim 6 being in the form of a powder, obtainable by absorbing the blend of claim 6 and/or the emulsion or cream of claim 7 onto a carrier, adding a gellable binder, inducing gelling of the carrier, and drying and grinding the gel.

9. Delivery form as described in claims 6-8, wherein the bioactive agent is an immunoglobulin preparation, such as an antiserum or polyclonal antibodies, for example in milk, eggs, serum or other animal tissues, or monoclonal antibodies.

10. Delivery form as claimed in claim 9, wherein the immunoglobulins are directed against bacterial, protozoan or viral fish and shellfish pathogens, such as Vibrio anguillarum.

11. Delivery form as claimed in claims 6-10, wherein the oil is an edible fish or vegetable oil.

12. Delivery form as claimed in claims 6-11, further comprising one or more preservatives, for example selected from the group consisting of benzoic acid, sorbic acid, propionic acid, or their salts, and/or antibacterial agents, such as protamine sulphate or rosemary extract.

13. Delivery form as claimed in claims 7-12, in which the water has been acidified, for example by means of ascorbic acid, acetic acid, acetic acid, lactic acid or citric acid.

14. Delivery form as claimed in any one of the claims 6-13 further comprising one or more anti-foaming agents, for example a silicon polymer.

15. Delivery form as claimed in any one of the claims 6-14 further comprising one or more oxygen removal agents, for example selected from the group consisting of glucose-oxidase, ascorbic acid or salts thereof.

16. Delivery form as claimed in any one of the claims 6-15 wherein the emulsifier is selected from the group consisting of lecithin, polyglycerol fatty acid, sorbitan fatty acid, sodium oleate, polyoxyethlene fatty acid ester, polyoxyethylene fatty glyceride, polyoxyethylene fat, and anhydrous maltose or combinations of one or more of these.

17. Delivery form as claimed in any one of the claims 6-16 further comprising one or more anti-oxidation agents, for example selected from the group consisting of butylated hydroxyanisole (BHA), butylated hydroxytoluene, (BHT),L-tocopherol, propyl gallate, carotinoids, and vitamin C palmitate.

18. Method of preparing a delivery form as claimed in claims 6 and 9-17, comprising blending the constituents a), b) and c), optionally together with other additives defined in claims 9-15.

19. Method of preparing a delivery form as claimed in claims 7 and 9-17, comprising blending the constituents a), b) and c), optionally together with other additives defined in claims 9-17, and emulsifying the so obtained blend in water to obtain an emulsion or cream.

20. Method of preparing a delivery form as claimed in claims 8-17, comprising blending the constituents a), b) and c), optionally together with other additives defined in claims 9-17, optionally emulsifying the so obtained blend in water to obtain an emulsion or cream, adsorbing the emulsion or cream onto a carrier material, adding a gellable binder, gelling the binder material and drying and grinding the gel.

21. Preparation for oral uptake for treatment and/or prophylaxis of animals, in particular fish and shellfish, with a bio-active agent, comprising small aquatic animals in particular zooplankton, to which the bio-active agent was administered by means of a delivery form as claimed in claims 6-17, optionally in the presence of a suitable excipient.

22. Preparation as claimed in claim 21, wherein the treatment consists of passive immunisation of fish and shellfish larvae and the bioactive agent comprises immunoglobulins.

23. Preparation as claimed in claim 22 wherein the immunoglobulins are directed against Vibrio anguillarum.

24. Use of small aquatic animals as claimed in claims 1-4 for preparing a preparation as claimed in claims 21, 22 and 23.

25. Process for preparing small aquatic animals as claimed in the claims 1-4, comprising feeding the small aquatic animals, such as zooplankton, with a delivery form according to any one of the claims 6-17.
